(19) Europäisches Patentamt
European Patent Office
Office européen des brevets

(11) Publication number: 0 014 985 A1

(12) EUROPEAN PATENT APPLICATION

(21) Application number: 80100813.7

(22) Date of filing: 18.02.80

(51) Int. Cl.³: C 07 C 85/08
C 07 C 87/06, C 07 C 87/36
B 01 J 27/04

(30) Priority: 21.02.79 US 13340

(43) Date of publication of application: 03.09.80 Bulletin 80 18

(84) Designated Contracting States: BE DE GB IT

(71) Applicant: UNIROYAL, INC.
1230 Avenue of the Americas Rockefeller Center
New York, New York 10020(US)

(72) Inventor: Malz Jr., Russel E.
261 Wedgewood Drive
Naugatuck, New Haven Connecticut(US)

(72) Inventor: Greenfield, Harold
85 Crestview Drive
Watertown, Litchfield Connecticut(US)

(74) Representative: Bühling, Gerhard, Dipl.-Chem. et al.
Patentanwaltsbüro Tiedtke-Bühling-Kinne
Grupe-Pellmann Bavariaring 4
D-8000 München 2(DE)

# (54) Trialkylamine preparation by reductive alkylation of dialkylamines with ketones.

(57) Trialkylamines are made by the action of hydrogen on a dialkylamine and a ketone in the presence of a metal sulfide catalyst. E.g., hydrogenation of a mixture of dibutylamine and cyclohexanone, using platinum sulfide catalyst, produces N,N-dibutylcyclohexylamine.

EP 0 014 985 A1

Croydon Printing Company Ltd.

This invention relates to a method of making trialkylamines.

There are several references that describe the reductive alkylation of dialkylamines with ketones to produce trialkylamines. However, we know of no prior art on the use of metal sulfide catalysts for this reaction. Schmerling, in U.S. 3,247,209 (April 19, 1966), has described the use of a sulfided platinum catalyst for the reductive alkylation of secondary alicyclic amines in which the nitrogen atom is part of a heterocyclic ring, specifically, 1,2,3,4-tetrahydroquinolines, with ketones.

This invention is concerned with the use of metal sulfide catalysts, such as the sulfides of iron, cobalt, nickel, ruthenium, rhodium, palladium, osmium, iridium, platinum, molybdenum, tungsten and rhenium, for the reductive alkylation of dialkylamines with ketones.

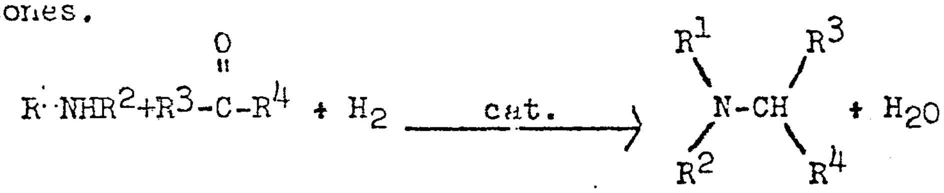


I

$R^1$ and $R^2$ may be the same or different and may be alkyl groups of from 1 to 18 carbon atoms or cycloalkyl groups of from 5 to 8 carbon atoms.

$R^3$ and $R^4$ may be the same or different and may be alkyl groups of from 1 to 17 carbon atoms or $R^3$ and $R^4$ taken together may form a polymethylene group having 4 to 5 carbon atoms or an alkyl substituted polymethylene group having 6 to 9 carbon atoms.

Examples of useful dialkylamines are dimethylamine, diethylamine, dipropylamine, diisopropylamine, dibutylamine, diisobutylamine, di-sec-butylamine, di-

amylamine, diisoamylamine, dihexylamine, dicyclohexylamine, diheptylamine, dioctylamine, bis(1-methylheptyl) amine, 2,2'-diethyldihexylamine, didecylamine, didodecylamine, N-methylbutylamine, N-ethylbutylamine, N-methylcyclohexylamine, N-ethylcyclohexylamine, N-isopropylcyclohexylamine, N-methyloctadecylamine, N-methylcyclodecylamine N,N'-diethylethylenediamine, N,N-dimethyl-N'-ethylethylenediamine, N,N,N'-triethylethylenediamine, N,N,N'-trimethylethylenediamine, N,N'-diethyl-1,3-propanediamine, N,N'-dimethyl-1,6-hexanediamine, etc.

Examples of useful ketones are acetone, 2-butanone, dihexyl ketone, 2,6-dimethyl-3-heptanone, 2,6-dimethyl-4-heptanone, 3,5-dimethyl-4-heptanone, 2,4-dimethyl-3-pentanone, 4,4-dimethyl-2-pentanone, 2-heptanone, 3-heptanone, 4-heptanone, 2-hexanone, 3-hexanone, mesityl oxide, 4-methyl-3-heptanone, 5-methyl-2-hexanone, 3-methyl-2-pentanone, 10-nonadecanone, 2-nonanone, 5-nonanone, 2-octanone, 3-octanone, 4-octanone, 2-pentanone, 3-pentanone, 2-undecanone, 6-undecanone, isophorone, cyclohexanone, cyclopentanone, 3-methylcyclohexanone, 4-t-butylcyclohexanone, etc.

The hydrogenation process may be carried out in the absence of any solvent medium, or in the presence of an inert organic solvent, including, for example, aliphatic or cycloaliphatic alcohols, such as methyl alcohol, ethyl alcohol, propyl alcohol, isopropyl alcohol, butyl alcohol, isobutyl alcohol, sec-butyl alcohol, tert-butyl alcohol, amyl alcohol, isoamyl alcohol, tert-amyl alcohol, hexyl alcohol, cyclohexanol, octyl alcohol, 2-ethylhexanol, decyl alcohol, dodecanol,

tetradecanol, hexadecanol, octadecanol, ethylene glycol, propylene glycol, glycerol, etc; ethers, such as diethyl ether, dipropyl ether, diisopropyl ether, methyl butyl ether, ethyl butyl ether, dibutyl ether, diamyl ether, diisoamyl ether, dihexyl ether, ethylene glycol dimethyl ether, ethylene glycol diethyl ether, ethylene glycol dibutyl ether, 1,3-dioxolane, 1,4-dioxane, tetrahydrofuran; alcohol-ethers, such as diethylene glycol, triethylene glycol, tetraethylene glycol, dipropylene glycol, tetraethylene glycol, dipropylene glycol, 2-butoxyethanol, tetrahydrofurfuryl alcohol, etc; esters, such as methyl acetate, ethyl acetate, propyl acetate, isopropyl acetate, butyl acetate, isobutyl acetate, methyl amyl acetate, 2-ethylhexyl acetate, diethyl succinate, etc; nitriles, such as acetonitrile, propionitrile, butyronitrile, etc; amides, such as formamide, acetamide, propionamide, N,N-dimethylformamide, N,N-diethylformamide, N,N-dimethylacetamide, N,N-diethylacetamide, etc; hydrocarbons, such as hexane, 2-methylpentane, 3-methylpentane, 2,2-dimethylbutane, 2,3-dimethylbutane, cyclohexane, heptane, 3-methylhexane, octane, 2,2,4-trimethylpentane, 2,3,4-trimethylpentane, decane, decalin, benzene, toluene, xylenes, etc. Preferred solvents are the aliphatic alcohols; most preferred are methyl alcohol and isopropyl alcohol.

The metal sulfide catalysts are important in this invention because they make the desired hydrogenation possible with little of the side reaction of reduction of ketones to alcohols commonly found with such non-sulfided catalysts as platinum, palladium, nickel, and

copper chromite. In addition, they are relatively insensitive to poisons that severely inhibit these other catalysts. Preferred catalysts are the sulfides of platinum, palladium, rhodium, ruthenium, cobalt and nickel. Most preferred are the sulfides of platinum, rhodium and ruthenium.

The catalyst may be prepared in situ or added to the reaction mixture after prior preparation and isolation. Commercial catalysts are available. The catalyst may be prepared and used as a bulk powder or supported on a suitable carrier, such as carbon or alumina. The catalyst may be prepared and used as a powder for liquid phase slurry and for vapor phase fluidized reactions, or as a pellet or granule for liquid or vapor phase fixed bed operations.

The reactions may be carried out in either batch or continuous systems, with either tank or pipe-line type reactors, in the liquid phase with either slurry or fixed bed catalysts, or in the vapor phase with either fluidized or fixed bed catalysts, operating in a manner well known to those skilled in the art.

The hydrogenation may be carried out under conventional reductive alkylation conditions, at temperatures ranging from room temperature or below, for example, 5° or less, to over 200°, for example, 250°, or other temperatures as high as the stability of the reactants and products will permit, and at pressures ranging from atmospheric pressure or below to pressures as high as economically practical, for example, up to 10,000 psig or more. Preferred is a temperature range from 20° to

220° (most preferably 90° to 200°) and a somewhat elevated pressure of, for example, 10 psig, up to, for instance, 5000 psig, usually 100 to 2500 psig. The conditions may be varied to provide an optimum economic combination of temperature, pressure, catalyst level, and cycle time for any given starting material and catalyst.

Example 1 illustrates the general applicability and utility of metal sulfide catalysts for the reductive alkylation of a dialkylamine, dibutylamine, with a ketone cyclohexanone, to produce a trialkylamine, N,N-dibutylcyclohexylamine. These results are summarized in Table 1.

Examples 2, 3, and 4A illustrate the use of a platinum sulfide catalyst for the preparation of trialkylamines by the reductive alkylation of a dialkylamine, dibutylamine, with a variety of ketones, acetone, methyl ethyl ketone, and methyl isobutyl ketone.

A comparison of examples 4A and 4B illustrates the superior performance of a platinum sulfide as compared to a palladium catalyst for the reductive alkylation of dibutylamine with methyl isobutyl ketone. The metal sulfide catalyst has better selectivity, as indicated by the higher yield of desired trialkylamine, N,N-dibutyl-1,3-dimethylbutylamine (60 vs 54%).

Example 5, the results of which are summarized in Table 2, compares a platinum sulfide catalyst with a non-sulfided platinum and a non-sulfided palladium catalyst for the reductive alkylation of a secondary

amine, N-ethylcyclohexylamine, with a ketone, cyclohexanone, to produce the tertiary amine, N-ethyldicyclohexylamine.

Platinum gave a very poor conversion of the starting secondary amine (27%) and a correspondingly low yield of the tertiary amine product (22%). The undesired reduction of ketone to alcohol was much more pronounced with the platinum than with the platinum sulfide catalyst. The platinum sulfide compared to the palladium resulted in a higher conversion of starting amine (52 vs 44%), and a higher yield of tertiary amine (42 vs 30%).

Example 1. N,N-Dibutylcyclohexylamine by reductive alkylation of dibutylamine with cyclohexanone.

A. To a 1-liter Magne Drive (trademark) autoclave were added 64.6 g (0.50 mole) of dibutylamine, 250 ml (ca. 2.4 mole) of cyclohexanone, and 3.5 g of 5% platinum sulfide on carbon. The autoclave was sealed, purged first with nitrogen and then with hydrogen, and hydrogen added to a pressure of 700 psig. The reaction mixture was heated with agitation for 10.3 hr at 45-50° and 500-800 psig, with little or no gas absorption in the last 0.8 hr. The autoclave was cooled and depressurized, and the reaction product was removed. The catalyst was removed by filtration through Celite filter-aid. A pure sample of N,N-dibutylcyclohexylamine was obtained by preparative glpc of a portion of the filtrate. Anal. Calcd for $C_{14}H_{31}N$: MW, 211. Found by titration with 0.1 N perchloric acid in acetic

acid: 212. Analysis of the filtrate by quantitative glpc indicated the presence of 10.9 g (17% yield) of recovered dibutylamine, 89 g (84% yield, 101% based on converted dibutylamine) of dibutylcyclohexylamine, and 9% reduction of the excess cyclohexanone to cyclohexanol.

B. Example 1A was repeated for 0.7 hr at 105-110° and 500-700 psig. Treatment and analysis of the reaction product as in Example 1A indicated the presence of 100 g (95% yield) of N,N-dibutylcyclohexylamine and no detectable dibutylamine.

C. Example 1A was repeated with 3.5 g of a 5% rhodium sulfide on carbon catalyst for 3.8 hr at 95-100° and 500-800 psig. Treatment and analysis of the reaction product as in Example 1A indicated the presence of 105.5 g (100% yield) of N,N-dibutylcyclohexylamine and no detectable dibutylamine.

D. A bulk palladium sulfide catalyst was prepared by substantially dissolving 10.0 g of palladium chloride dihydrate in 1 liter of 0.3 N hydrochloric acid with stirring and then bubbling in gaseous hydrogen sulfide for 0.2 hr. The black precipitate was filtered, washed with 2 liters of distilled water, then with 500 ml of 2-propanol, and then with 500 ml of cyclohexanone.

Example 1A was repeated with the above palladium sulfide for 7.0 hr at 135-145° and 500-800 psig. Treatment and analysis of the reaction product as in Example 1A indicated the presence of 99 g (94% yield) of N,N-dibutylcyclohexylamine and no detectable dibutylamine.

E. A bulk ruthenium sulfide catalyst was prepared by bubbling gaseous hydrogen sulfide for 1 hr

into a solution of 10.0 g of ruthenium trichloride hydrate ($RuCl_3 \cdot$ 1-3 $H_2O$) in 1 liter of distilled water. The black precipitate was filtered, washed with 2 liters of distilled water, then with 500 ml of 2-propanol, and then with 1 liter of cyclohexanone.

Example 1A was repeated with the above ruthenium sulfide for 3.3 hr at 75-80° and 500-800 psig. Treatment and analysis of the reaction product as in Example 1A indicated the presence of 102 g (96% yield) of N,N-dibutylcyclohexylamine and no detectable dibutylamine.

F. Example 1A was repeated with 7.0 g of a 20% molybdenum sulfide on alumina catalyst (Girdler T-318) for 4.3 hr at 240-250° and 800-1000 psig. Treatment and analysis of the reaction product as in Example 1A indicated the presence of 94 g (89% yield) of N,N-dibutylcyclohexylamine, no detectable dibutylamine, and 11% reduction of the excess cyclohexanon to cyclohexanol.

Table 1[a] Reductive alkylation of dibutylamine with cyclohexa

| Example No. | Catalyst | Temp., °C | Pressure, psig | Time, hr | Yield 3° amir mole % |
|---|---|---|---|---|---|
| 1A | Pt sulfide | 45-50 | 500-800 | 10.3[c] | 84[d] |
| 1B | Pt sulfide | 105-110 | 500-700 | 0.7 | 95 |
| 1C | Rh sulfide | 95-100 | 500-800 | 3.8 | 100 |
| 1D | Pd sulfide | 135-145 | 500-800 | 7.0 | 94 |
| 1E | Ru sulfide | 75-80 | 500-800 | 3.3 | 96 |
| 1F | Mo sulfide | 240-250 | 800-1000 | 4.3 | 89 |

a. Each experiment was run with 64.6 g (0.50 mole) of dibutylamine and 250 ml (ca. 2.4 mole) of cyclohexanone. There was no detectable dibutylamine except in Example 1/

b. N,N-dibutylcyclohexylamine.

c. Little or no gas absorption in last 0.8 hr.

d. 17% recovery of dibutylamine; 101% yield of N,N-dibutylcyclohexylamine based on converted dibutylamine.

Example 2. N,N-Dibutylisopropylamine by reductive alkylation of dibutylamine with acetone.

To a 1-liter Magne Drive autoclave were added 64.6 g (0.50 mole) of dibutylamine, 250 ml (ca. 3.4 mole) of acetone, and 3.5 g of a 5% platinum sulfide on carbon catalyst. The autoclave was sealed, purged first with nitrogen and then with hydrogen, and hydrogen added to a pressure of 500 psig. The reaction mixture was heated with agitation for 2.0 hr at 90-100° and 500-800 psig, with little or no gas absorption in the last 0.3 hr. The autoclave was cooled and depressurized, and the reaction product was removed. The catalyst was removed by filtration through Celite filter-aid. A portion of the N,N-dibutylisopropylamine was distilled at 191-192° Anal. Calcd for $C_{11}H_{25}N$: MW, 171. Found by titration with 0.1 N perchloric acid in acetic acid: 172.

Example 3. N,N-Dibutyl-sec-butylamine by reductive alkylation of dibutylamine with methyl ethyl ketone.

To a 1-liter Magne Drive autoclave were added 64.6 g 0.50 mole of dibutylamine, 250 ml (ca. 2.8 mole) of methyl ethyl ketone, and 3.5 g of a 5% platinum sulfide on carbon catalyst. The autoclave was sealed, purged first with nitrogen and then with hydrogen, and hydrogen added to a pressure of 500 psig. The reaction mixture was heated with agitation for 1.6 hr at 140-150° and 500-800 psig. The autoclave was cooled and depressurized, and

the reaction product was removed. The catalyst was removed by filtration through Celite filter-aid. A pure sample of N,N-dibutyl-sec-butylamine was obtained by preparative hplc of a portion of the concentrated filtrate. Anal. Calcd for $C_{12}H_{27}N$: C, 77.76; H, 14.68; N, 7.56. Found: C,77.93; H, 14.93; N, 7.62.

Example 4. N,N-dibutyl-1,3-dimethylbutylamine by reductive alkylation of dibutylamine with methyl isobutyl ketone

A. To a 1-liter Magne Drive autoclave were added 64.6 g (0.50 mole) of dibutylamine, 250 ml (ca. 2.0 mole) of methyl isobutyl ketone, and 3.5 g of a 5% platinum sulfide on carbon catalyst. The autoclave was sealed, purged first with nitrogen and then with hydrogen and hydrogen added to a pressure of 500 psig. The reaction mixture was heated with agitation for 5.0 hr at 200° and 500-800 psig. The autoclave was cooled and depressurized, and the reaction product was removed. The catalyst was removed by filtration through Celite filter-aid. A pure sample of N,N-dibutyl-1,3-dimethylbutylamine was obtained by preparative hplc of a portion of the distilled filtrate. Anal. Calcd for $C_{14}H_{31}N$: C, 78.79; H, 14.64; N, 6.56. Found: C, 78.82; H, 14.58; N, 6.55. A quantitative glpc analysis of the filtrate indicated the presence of 64 g (60% yield) of N,N-dibutyl-1,3-dimethylbutylamine and no detectable dibutylamine.

B. (Comparison experiment outside the invention.) To a 1.7-liter rocking autoclave were added 129.2 g (1.00 mole) of dibutylamine, 430 ml (ca. 3.4 mole) of methyl isobutyl ketone, and 12.0 g of a 5% palladium on carbon

catalyst. The autoclave was sealed, purged first with nitrogen and then with hydrogen, and hydrogen added to a pressure of 600 psig. The reaction mixture was heated with agitation for 2.6 hr at 190-205° and 600-800 psig, with little or no gas absorption in the last 0.7 hr. The autoclave was cooled and depressurized, and the reaction product was removed. The catalyst was removed by filtration through Celite filter-aid. A quantitative glpc analysis of the filtrate indicated the presence of 114 g (54% yield) of N,N-dibutyl-1,3-dimethylbutylamine and no detectable dibutylamine.

Example 5. N-Ethyldicyclohexylamine by reductive alkylation of N-ethylcyclohexylamine with cyclohexanone.

A. To a 1-liter Magne Drive autoclave were added 63.6 g (0.50 mole) of N-ethylcyclohexylamine, 54.0 g (0.55 mole) of cyclohexanone, 115 ml of methanol, and 0.5 g of a 5% platinum sulfide on carbon catalyst. The autoclave was sealed, purged first with nitrogen and then with hydrogen, and hydrogen added to a pressure of 500 psig. The reaction mixture was heated with agitation for 7.5 hr at 160° and 600-775 psig. The autoclave was cooled and depressurized, and the reaction product was removed. The catalyst was removed by filtration through Celite filter-aid. A quantitative glpc analysis of the filtrate indicated the presence of 30.3 g (48% yield) of recovered N-ethylcyclohexylamine, 44.5 g (42% yield, 81% based on converted N-ethylcyclohexylamine) of N-ethyldicyclohexylamine, 8.6 g (16% yield) of recovered cyclohexanone, and 19.0 g (35% yield based on

cyclohexanone) of cyclohexanol.

B. (Comparison experiment outside the invention.) Example 5A was repeated with 0.5 g of 5% platinum on carbon as catalyst for 7.5 hr at 160° and 600-755 psig. Treatment and analysis of the reaction product as in Example 5A indicated the presence of 46.7 g (73% yield) of recovered N-ethylcyclohexylamine, 23.4 g (22% yield, 81% based on converted N-ethylcyclohexylamine) of N-ethyldicyclohexylamine, 8.6 g (16% yield) of recovered cyclohexanone, and 33.8 g (61% yield based on cyclohexanone) of cyclohexanol.

C. (Comparison experiment outside the invention.) Example 5B was repeated with 0.5 g of 5% palladium on carbon as catalyst for 7.5 hr at 160° and 615-760 psig. Treatment and analysis of the reaction product as in Example 5B indicated the presence of 35.7 g (56% yield) of recovered N-ethylcyclohexylamine, 31.7 g (30% yield, 68% based on converted N-ethylcyclohexylamine) of N-ethyldicyclohexylamine, 12.6 g (23% yield) of recovered cyclohexanone, and 18.7 g (35% yield based on cyclohexanone) of cyclohexanol.

Table 2. Reductive alkylation of N-ethylcyclohexylamine with cyclohexanone a/

| | | Yield, mole % | | | | |
|---|---|---|---|---|---|---|
| Example No. | Catalyst | Recovered 2° amine | 2° amine b/ actual | 2° amine b/ based on conv. | recovered cyclohexanone | cyclohexanol c/ |
| 5A | Pt sulfide | 48 | 42 | 81 | 16 | 35 |
| 5B | Pt | 73 | 22 | 81 | 16 | 61 |
| 5C | Pd | 56 | 30 | 68 | 23 | 35 |

a. Each experiment was run in a 1-liter Magne Drive autoclave with 63.6 g (0.50 mole) of N-ethylcyclohexylamine, 54.0 g (0.55 mole) of cyclohexanone, 115 ml of methanol and 0.5 g of a 5% supported on carbon catalyst for 7.5 hr at 160° and in the range of 600-760 psig.

b. N-ethyldicyclohexylamine.

c. Based on starting cyclohexanone.

What is claimed is:

1. A method of making a trialkylamine of the formula

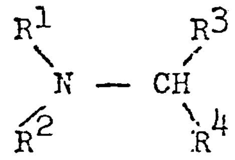

by reductive alkylation, characterized by bringing together a dialkylamine of the formula:

$$R^1NHR^2$$

a ketone of the formula:

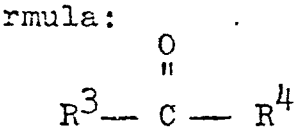

hydrogen, and a catalytic amount of a metal sulfide hydrogenation catalyst and thereafter recovering the resulting trialkylamine, the values of the R's in the said formulas being as follows:

$R^1$ and $R^2$ may be the same or different and may be alkyl groups of from 1 to 18 carbon atoms or cycloalkyl groups of from 5 to 8 carbon atoms;

$R^3$ and $R^4$ may be the same or different and may be alkyl groups of from 1 to 17 carbon atoms or $R^3$ and $R^4$ taken together may form a polymethylene group having 4 to 5 carbon atoms or an alkyl substituted polymethylene group having 6 to 9 carbon atoms.

2. A method as in claim 1 in which the said dialkylamine is dibutylamine or N-ethylcyclohexylamine.

3. A method as in claim 1 or claim 2 in which the said ketone is cyclohexanone, acetone, methyl ethyl ketone or methyl isobutyl ketone.

4. A method as in claim 1 in which the said catalyst is a sulfide of iron, cobalt, nickel, ruthenium, rhodium, palladium, osmium, iridium, platinum, molybdenum, tungsten or rhenium.

5. A method as in claim 1, claim 2 or claim 3 in which the said catalyst is platinum sulfide.

6. A method as in claim 1 carried out at a temperature of from 5° to 250°C and a pressure of from atmospheric to 10,000 psig.

0014985

European Patent Office

# EUROPEAN SEARCH REPORT

Application number

EP 80 10 0813

## DOCUMENTS CONSIDERED TO BE RELEVANT

| Category | Citation of document with indication, where appropriate, of relevant passages | Relevant to claim |
|---|---|---|
| X | GB - A - 1 064 958 (UNITED STATES RUBBER) * claims 1 to 4 * -- | 1,4-6 |
| | DE - B - 1 518 402 (UNIROYAL) * claim 1 * -- | 1 |
| A | GB - A - 1 117 332 (UNIVERSAL OIL PRODUCTS) -- | |
| D,A | US - A - 3 247 209 (UNIVERSAL OIL PRODUCTS) -- | |

CLASSIFICATION OF THE APPLICATION (Int. Cl.3)

C 07 C 85/08
C 07 C 87/06
C 07 C 87/36
B 01 J 27/04

TECHNICAL FIELDS SEARCHED (Int Cl.3)

C 07 C 85/08
C 07 C 87/06
C 07 C 87/36

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 14-05-1980 | FROELICH |